# EUROPÄISCHE PATENTSCHRIFT

(11) **EP 3 048 992 B1**
(45) Veröffentlichungstag und Bekanntmachung des Hinweises auf die Patenterteilung: **27.03.2019**
(21) Anmeldenummer: 14767007.9
(22) Anmeldetag: 18.09.2014
(51) Int. Cl.: A61B 17/02

(54) **CHIRURGISCHER RETRAKTOR MIT ABNEHMBAREM BETÄTIGUNGSELEMENT**
SURGICAL RETRACTOR WITH REMOVABLE ACTUATING ELEMENT
RÉTRACTEUR CHIRURGICAL POURVU D'UN ÉLÉMENT D'ACTIONNEMENT AMOVIBLE

(30) Priorität: 27.09.2013 DE 102013110717
(43) Veröffentlichungstag der Anmeldung: 03.08.2016
(73) Patentinhaber: Aesculap AG, 78532 Tuttlingen (DE)
(72) Erfinder: VOGTHERR, Robert, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2014/069905
(87) Internationale Veröffentlichungsnummer: WO 2015/044026

(56) Entgegenhaltungen:
- WO-A1-2009/124244
- WO-A1-2014/146824
- US-A- 4 747 394
- US-A- 5 052 373

## Beschreibung

Die vorliegende Erfindung betrifft einen Retraktor (mit abnehmbarer Retraktorkurbel) gemäß dem Oberbegriff des Patentanspruchs 1.

### Hintergrund der Erfindung

Bei vielen herzchirurgischen Eingriffen muss das Sternum des Patienten in Längsrichtung durchtrennt und anschließend die Sternumhälften mit den daran anschließenden Rippen auseinander gespreizt werden, um dem Operateur das Arbeiten am Herz zu ermöglichen. Zum Spreizen dieser Operationsöffnung werden Retraktoren (auch Sperrer oder Spreizer genannt) eingesetzt.

Der Operateur arbeitet teilweise mehrere Stunden in diesem von einem Retraktor offen gehaltenen Operationsgebiet. Damit dieser Retraktor selbst nicht störend oder hinderlich ist, sollte er so flach wie möglich am Patient anliegen. Der Operateur legt erfahrungsgemäß oftmals auch seine Handballen zum Schonen der Handgelenke auf dem Retraktorrahmen ab. Gerade kleine, eckige oder andersweitig unangenehme hervorstehende Konturen stehen dem im Wege.

Des Weiteren darf der Retraktor über keinerlei Konturen verfügen, die ein Ein- oder Festhängen von chirurgischen Fäden und Nahtmaterial verursachen würden. Alle Geometrien sollen daher glatt und eben ausgebildet sein.

Der Rahmen des Retraktors, in der Regel bestehend aus einer oder mehrerer Zahnstangen, die einen längenverstellbaren Querriegel bilden, zwei Valven-Armen, die an den Längsenden des Querriegels angeformt oder dort fixiert sind und einem Antriebskasten, der am Querriegel montiert ist, stellen hierbei kein größeres Dimensionierungsproblem dar. Ein solcher Retraktor ist bei allen bekannten Ausführungsformen relativ flach.

Das einzige Bauteil, welches immer und bei allen auf dem Markt vertretenen Retraktoren von den Operateuren als störend empfunden wird, ist die Antriebskurbel, über welche die Längenverstellung des Querriegels für ein Aufspreizen des Sternums manuell vorgenommen wird. Sie muss groß genug sein, dass sie gut gegriffen werden kann und einigermaßen ergonomisch in der Hand liegt. Damit baut sie mehrere Zentimeter über dem flachen Antriebskasten auf und ist das einzige Bauteil, das aus der flachen Silhouette des Retraktors herausragt.

### Stand der Technik

Da eine sehr kleine, platzsparende Kurbel zwar weniger störend wäre, aber zur Handhabung nicht praktikabel ist, ist eine abnehmbare Kurbel die bisher beste technische Lösung. In der EP 1 471 831 B1 wird ein Antrieb eines Sperrers der vorliegenden Gattung offenbart, bei dem die Kurbel vom Retraktor eigenen Antrieb selbst abnehmbar ist. Es handelt sich hierbei um einen Antrieb, bei dem ein Vierkant aus einem Antriebskasten herausragt, der am Retraktor montiert ist. Auf diesen Vierkant wird eine Kurbel mit einem entsprechend passenden Innenvierkant formschlüssig aufgesteckt. Eventuell verfügen die beiden Elemente auch über ein Rastelement, beispielsweise ein Kugeldruckstück und eine Gegenform als Rastposition zur Sicherung des zusammengesteckten Zustands.

Die abnehmbare Kurbel gemäß der EP1 471 831 B1 weist jedoch den Nachteil auf, dass sie die Störkontur des Antriebs im abgenommenen Zustand zwar verringert, aber nicht völlig beseitigt. Ein Vierkant oder ein beliebiger anders geformter Bolzen bleibt immer bestehen und kann vom Operateur als unzweckmäßig empfunden werden. Insbesondere ist die Kurbel im zusammengesteckten Zustand in der Regel nicht sicher auf dem Vierkant eines solchen Antriebs adaptiert. Die Steckverbindung geschieht nur formschlüssig in einer Ebene. Dem Abnehmen der Kurbel vom Antrieb (vom Vierkant), also dem Umkehren des Steckvorgangs, wird entweder gar nicht oder eventuell durch ein Kugeldruckstück oder ein ähnliches Rastelement nur in sehr geringer Weise und in diesem Fall kraftschlüssig entgegengewirkt. Dieses Rastelement besitzt auf jeden Fall keine eigenständige Betätigung, d.h. es wird mit dem Abziehen der Kurbel vom Kurbelzapfen automatisch betätigt und stellt daher
einen nur geringen Widerstand dar. Daher kann die Kurbel systembedingt genauso leicht wie sie aufgesteckt wird auch wieder abgezogen werden.

Der Operateur wird in der Anwendung jedoch nicht nur Radial-, sondern auch unbeabsichtigte Druck- und Zugkräfte in Längsrichtung der Vierkantachse an der Kurbel ausüben. Dadurch kann sich die Kurbel bei nicht ausreichender Sicherung des zusammengesteckten Zustands ungewollt lösen und die Arbeit mit dem Retraktor erschweren.

Grundsätzlich bestünde natürlich die Möglichkeit, die Kurbel beispielsweise mittels einer Wellenmutter auf dem Vierkant zu sichern. Auch könnten Splinte oder dergleichen Sicherungselemente gegen ein unbeabsichtigtes Abziehen der Kurbel vom Vierkant (Kurbelzapfen) angeordnet sein. Jedoch braucht es bei dieser Form der Axialsicherung immer eine zweite Hand und/oder einen zweiten Montage-/ Demontageschritt, der die Funktionalität der Retraktors herabsetzt.

Die US 4747394 offenbart einen Retraktor mit abnehmbaren, formschlüssigen Betätigungselement und damit die Präambel des unabhängigen Anspruchs.

### Kurzbeschreibung der Erfindung

Die Aufgabe der vorliegende Erfindung besteht demnach darin, einen Retraktor bereit zu stellen, der eine gegenüber dem vorstehend genannten Stand der Technik höhere Funktionalität aufweist.

Ein Ziel der Erfindung ist es dabei, die Handhabbarkeit des Retraktors im Einsatz zu steigern.

Ein weiteres Ziel der Erfindung ist es, die Betriebssicherheit des Retraktors zu erhöhen. Insbesondere hat die Erfindung zum Ziel, eine von einem Retraktor-Antrieb abnehmbare Kurbel mit Betätigungselement bereit zu stellen, welche sich nicht ungewollt oder unbeabsichtigt vom Antrieb lösen kann und welche im abgenommenen Zustand einen wirklich ebenen Antriebskasten / Antriebsgehäuse ohne hervorstehende Konturen generiert.

Im Ergebnis soll der erfindungsgemäße Retraktor dem Operateur keine Hindernisse entgegenstellen, sodass ein Ein- oder Festhängen von chirurgischen Fäden und Nahtmaterial im Bereich des Antriebs weitestgehend ausgeschlossen werden kann.

Die vorstehende Aufgabe wird durch einen Retraktor mit den Merkmalen des Anspruchs 1 gelöst. Vorteilhafte Ausgestaltungen der Erfindung sind Gegenstand der Unteransprüche.

Der Grundgedanke der Erfindung besteht darin, die Funktionalität des Retraktors zunächst dadurch zu erhöhen, indem das Getriebe-Eingangselement des am Retraktor fest montierten Antriebs in das Antriebsgehäuse zurückversetzt wird, um keinen oder nur einen vernachlässigbaren Überstand gegenüber der Gehäuseaußenseite zu bewirken. Erzielt wird dies vorzugsweise dadurch, indem das in Übereinstimmung mit dem Stand der Technik als Drehbauteil ausgebildete und gelagerte Getriebe-Eingangselement an seiner nach außerhalb des Getriebegehäuses exponierten Seite mit zumindest einer Aussparung ausgebildet oder versehen ist, die für eine Drehmomentübertragung von dem Betätigungselement, z.B. der Kurbel auf das Getriebe-Eingangsbauteil angepasst ist, wohingegen die abnehmbare Kurbel mit zumindest einem Vorsprung ausgebildet oder versehen ist, der in die zumindest eine Aussparung drehfest einführbar ist.

Dadurch wird die drehfeste Verbindung zwischen Kurbel und Getriebe-Eingangselement quasi aus der Kurbel heraus in das Getriebe-Eingangselement und damit in das Antriebsgehäuse verlagert. In anderen Worten ausgedrückt, wird der im Stand der Technik bekannte Vierkant oder das andersartig geformte Drehmoment-Übertragungsmittel nicht am Getriebe-Eingangselement angeordnet und verbleibt dort ggf. störend auch nach Abnehmen der Kurbel, sondern ist nunmehr an der Kurbel vorgesehen und wird daher zusammen mit dieser abgenommen.

Um ein ausreichendes Drehmoment übertragen zukönnen, ist es erforderlich, die Kraftangriffsflächen am Getriebe-Eingangsbauteil möglichst groß zu gestalten, sodass die Flächenpressung nicht das verwendete Material überfordert oder alternativ hierzu das Drehmoment-Übertragungsmittel so zu gestalten, dass ein großer Hebelarm am Getriebe-Eingangselement entsteht.

Bevorzugt ist demnach die Bereitstellung des Retraktors mit wenigstens zwei Retraktorarmen, deren proximale Endabschnitte an den sich gegenüberliegenden Enden eines längenverstellbaren Querriegels zur Einstellung des Querabstands zwischen den Retraktorarmen fixiert oder einstückig daran angeformt sind und der Antriebseinheit, deren Gehäuse am Querriegel montiert ist und deren Getriebe (Zahnradgetriebe) mit einem Längenverstellmechanismus des Querriegels wirkgekoppelt ist, um mittels des separaten Betätigungselements (Kurbel) manuell betätigt zu werden, das mit dem in das Gehäuse bewegbar, vorzugsweise drehbar, eingesetzten Getriebe-Eingangselement in Eingriff bringbar ist, das hierfür an wenigstens einer Gehäuseaußenseite von außen zugänglich ist. Das Getriebe-Eingangselement ist, wie vorstehend bereits ausgeführt wurde, im Wesentlichen bündig (ggf. mit vernachlässigbarem Überstand) mit der Gehäuseaußenseite oder bezüglich der Gehäuseaußenseite in das Gehäuseinnere sogar zurückgesetzt. Auf diese Weise entstehen keine Kanten oder dergleichen Hindernisse, an denen OP-Material wie Nahtfäden hängen bleiben kann. Die Oberfläche des Antriebsgehäuses wird durchgehend glatt, sodass auch die Handhabung des Retraktors verbessert werden kann.

Gemäß der vorliegenden Erfindung ist es vorgesehen, dass das Getriebe-Eingangselement drehbar im Antriebsgehäuse gehalten ist und an seiner nach außen hin exponierten Seite wenigstens eine Aussparung aufweist, die als ein Kraft-/Momenteinleitungsmittel für das Betätigungselement angepasst ist. Dadurch ragt kein Teil des Getriebe-Eingangselements aus dem Gehäuse (auch nicht temporär) vor. Die Aussparung kann dabei eine Form haben, die von einer Kreisform abweicht (z.B. Mehrkantprofil oder dergleichen), um einen Formschluss in Drehrichtung mit dem Betätigungselement einzugehen. In diesem Fall ist die zumindest eine Aussparung bezüglich der Drehbewegung zentral am Getriebe-Eingangselement angeordnet. Alternativ oder zusätzlich sind eine Anzahl (Mehrzahl) von im Umfangs- und/oder Radialrichtung des vorzugsweise kreisförmigen Getriebe-Eingangselements beabstandete Aussparungen ausgebildet, die als Kraft-/Momenteinleitungsmittel für das Betätigungselement vorgesehen sind und infolge ihres Abstands zueinander einen Hebelarm aufspannen. Dadurch können höhere Drehmomente in das Getriebe-Eingangselement eingeleitet werden und/oder die einzelnen Aussparungen können kleiner dimensioniert und weniger tief im Getriebe-Eingangselement ausgenommen sein. Dies wiederum ermöglicht es, das Getriebe-Eingangselement teller- oder scheibenförmig (flach bezüglich seines Durchmessers) auszubilden und so das Getriebegehäuse entsprechend schmal zu bauen. Dadurch wird die Handhabbarkeit des Retraktors weiter verbessert.
Vorzugsweise ist das Betätigungselement als eine Art Kurbel oder Ratsche ausgebildet mit einem Hebel, an dessen einem Endabschnitt ein Handgriff und an dessen anderem Ende ein Eingriffskopf (Kurbelkopf) vorgesehen ist mit wenigstens einem, vorzugsweise wenigstens zwei Vorsprüngen, die gleichzeitig in wenigstens zwei der Aussparungen einfügbar sind, um ein Drehmoment auf das Getriebe-Eingangselement zu übertragen.

Gemäß der vorliegenden Erfindung ist es vorgesehen, dass zumindest eine Aussparung auf Seiten der Antriebseinheit mit einem axial wirkenden Hinterschnitt ausgeformt ist und wenigstens ein Vorsprung aus Seiten des Betätigungselements mit einer axial wirkenden Rastkante ausgeformt ist, derart, dass bei Einfügen des wenigstens einen Vorsprungs in die zumindest eine Aussparung die Rastkante in den Hinterschnitt greift und somit ein unbeabsichtigtes Herausziehen des Vorsprungs aus der Aussparung ver-/behindert.
Vorzugsweise ist es vorgesehen, dass der zumindest eine Vorsprung mit der Rastkante in und entgegen der Rastrichtung (bezüglich der Drehrichtung des Getriebe-Eingangselements radial) bewegbar ist, um unabhängig von der Einfüg-/Abziehbewegung des Vorsprungs in/aus der Aussparung zumindest in eine Außereingriffsposition bezüglich des Hinterschnitts manuell überführbar zu sein. D.h. eine automatische Betätigung der Verrastung infolge des Abziehens des Betätigungselements vom Getriebe-Eingangselement, wie im Stand der Technik vorgesehen, wird erfindungsgemäß ausgeschlossen, da für das Freigeben der Verrastung eine unabhängige, separate manuelle Betätigung erforderlich ist. Dadurch wird ein unbeabsichtigtes Abziehen des Betätigungselements im Betrieb verhindert.
Die Erfindung wird nachstehend anhand eines bevorzugten Ausführungsbeispiels unter Bezugnahme auf die begleitenden Figuren näher erläutert, wobei auch auf nicht figürlich dargestellte alternative Ausgestaltungen hingewiesen wird.
Fig. 1 zeigt die Perspektivenansicht eines Retraktors gemäß einem bevorzugten Ausführungsbeispiel der vorliegenden Erfindung,
Fig. 2 zeigt in Vergrößerung eine Antriebseinheit des Retraktors gemäß Fig. 1,
Fig. 3 zeigt einen Querschnitt eines Betätigungselements, z.B. Kurbel, gemäß einem bevorzugten Ausführungsbeispiel der vorliegenden Erfindung in einer Rast- oder Eingriffsposition mit dem Getriebe-Eingangselement einer Retraktorseitigen Antriebseinheit und
Fig. 4 zeigt einen Querschnitt des Betätigungslements von Fig. 2 in einer manuell separat/individuell bewirkten Außer-Rastposition mit dem Getriebe-Eingangselement.

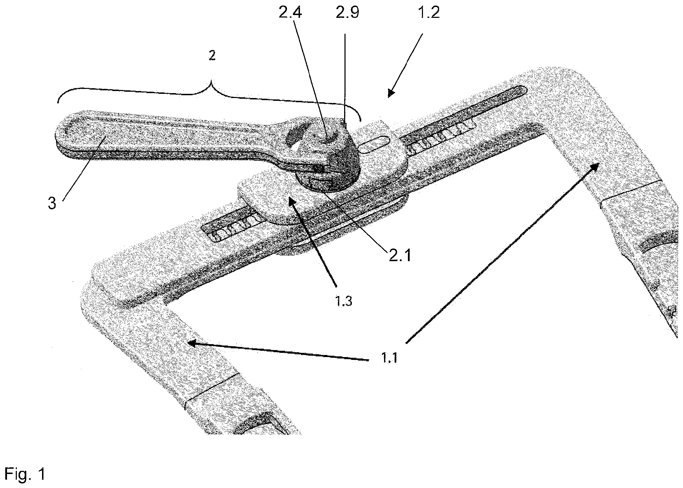

Gemäß der Fig. 1 besteht der erfindungsgemäße Retraktor 1 im Wesentlichen aus zwei Retraktor-Armen 1.1, die an ihren distalen freie Enden mit Patienten-Eingriffsmitteln (nicht weiter dargestellt) versehen oder versehbar sind und an ihren proximalen Enden mit einem längenverstellbaren, bzw. einen Längenverstellmechanismus aufweisenden Querriegel verbunden oder zu diesem ausgeformt sind. Im Konkreten bilden die zwei Retraktor-Arme 1.1 jeweils ein Winkelelement mit einem Patienten-Eingriffsschenkel, der den Retraktor-Arm darstellt und einem rechtwinklig hierzu sich erstreckenden Querriegelschenkel, der einen Teil des Querriegels darstellt. Wenigstens einer der Querriegelschenkel ist mit einer Stangenverzahnung (als Längenverstellmechanismus) ausgeformt, wobei die beiden Querriegelschenkel relativ zueinander längsverschiebar aneinander gehalten sind. Ferner ist in einem Mittenabschnitt des durch die beiden Querriegelschenkel gebildeten Querriegels eine Antriebseinheit 1. 2 montiert mit einem Getriebe (nicht weiter dargestellt), das auf die wenigstens eine Stangenverzahnung einwirkt und somit den einen Querriegelschenkel bezüglich des anderen Querriegelschenkels längsverschiebt. Das nicht dargestellte Getriebe ist in einem Antriebskasten oder
Gehäuse 1.3 untergebracht, der an dem Querriegelschenkel ohne Verzahnung fixiert ist.

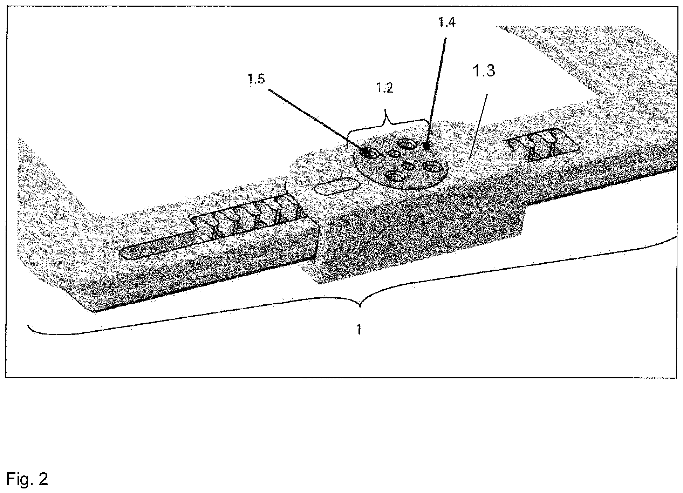

Die Antriebseinheit 1.2 des Retraktors 1 besitzt gemäß der Fig. 2 ein Getriebe-Eingangselement, vorliegend in Form einer (oberen/vorderen) Scheibe 1.4, die drehbar um ihre Mittenachse im Antriebskasten/Antriebsgehäuse 1.3 gehalten ist mit einer (Flach-) Seite, die zur Außenseite des Antriebskastens 1.3 exponiert/frei zugänglich ist und die mit dem Antriebskasten 1.3 zumindest an dieser Außenseite im Wesentlich bündig abschließt und aus diesem nicht oder nur vernachlässigbar hervorsteht. An der exponierten (kreisförmigen) Außenseite des Getriebe-Eingangselements 1.4 sind vorliegen eine Anzahl (vier) vorzusgweise gleichmäßig winkelbeabstandeter Aussparungen 1.5 in Form von (senkrecht zur Flachseite ausgerichteten) Bohrungen ausgeformt, welche vorliegend die Eckpunkte eines Quadrats auf der kreisförmigen Oberseite des Getriebe-Eingangselements 1.4 darstellen. Die Aussparungen 1.4 bilden ein Drehmoment-Eingangsmittel zur Drehmomentübertragung von einem Betätigungselement 2, nämlich Kurbel oder Ratsche, in das Getriebe-Eingangselement 1.4. Zu diesem Zweck spannen die Aussparungen 1.4 eine Art imaginärer Hebelarm auf, dessen Länge dem Abstand jeweils zweier diametral gegenüberliegender Aussparungen 1.4 entspricht.

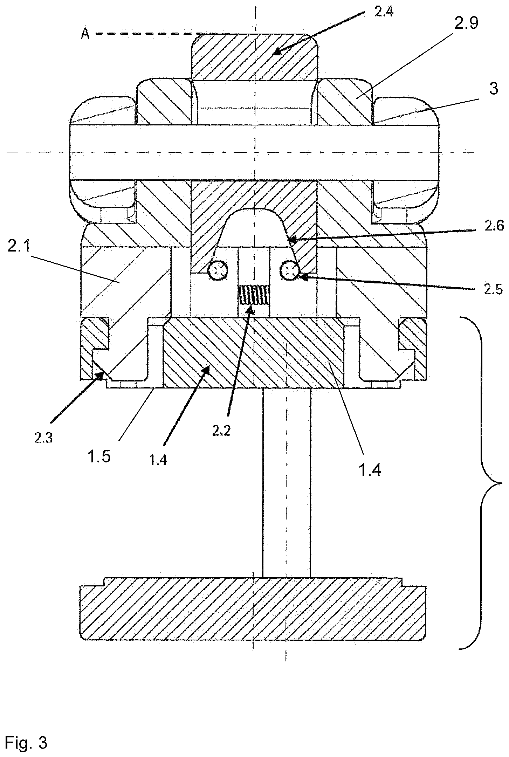

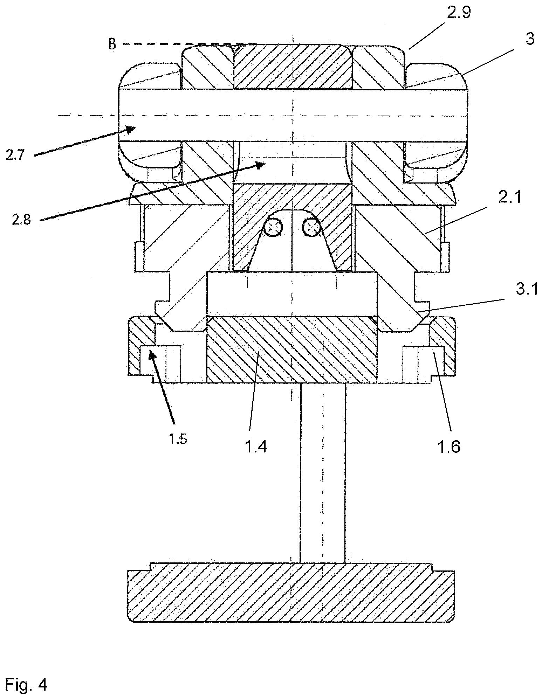

Im Gegenzug hat gemäß Fig. 1 das separat zu der Antriebseinheit 1.2 ausgebildete Betätigungselement 2 einen Griffhebel 3, an dessen einem Ende eine Elementkopf (nachfolgend Kurbelkopf) 2.9 angeordnet ist, der vorliegend gemäß der Fig. 3, 4 mit zwei Eingriffsvorsprüngen oder Bolzen (Eingriffselemente) 2.1 senkrecht zum Griffhebel 3 ausgebildet ist, die wahlweise in zwei diametral sich gegenüberliegende Aussparungen 1.5 eingeführt werden können, wie dies nachfolgend noch näher beschrieben wird. Auf diese Weise kann je nach Anzahl der Aussparungen 1.5 die Kurbel 2 in verschiedenen Winkelpositionen auf die Antriebseinheit 1.2 / das Getriebe-Eingangselement 1.4 gesteckt werden.

In der Fig. 1 ist der erfindungsgemäß Retraktor 1 mit aufgesetztem/aufgestecktem Betätigungselement/Kurbel 2 dargestellt, wobei in diesem Fall die Vorsprünge/Bolzen/Zapfen/etc. 2.1 der Kurbel 2 in die in Fig. 1 nicht sichtbaren Aussparungen 1.5 auf Seiten des Getriebe-Eingangselements 1.4 eingeführt sind, um ein Drehmoment auf das Getriebe-Eingangselment 1.4 zu übertragen. Dieser eingeführte Zustand ist in den Fig. 3 und 4 im Querschnitt gezeigt und wird nachfolgend näher beschrieben.

Jede (zumindest eine) Aussparung 1.5 verfügt gemäß der Fig. 3, 4 über einen in Axialrichtung der Aussparung 1.5 wirkenden Hinterschnitt 1.6, wohingegen die in die Aussparungen 1.5 einzufügenden Vorsprünge oder Bolzen 2.1 des Betätigungselements (Kurbel/Ratsche) 2 mit entsprechenden Rastkanten (hakenförmige Rastnasen) 3.1 ausgebildet sind, die mit den Hinterschnitten / Hinterschneidungen 1.6 mit Einführen der Vorsprünge 2.1 in die Aussparungen 1.5 in axial wirkenden Rasteingriff bringbar sind, um ein Herausziehen der Vorsprünge 2.1 aus den Aussparungen 1.5 zu blockieren.

Jede Rastkante/Rastnase 3.1 oder jeder, eine Rastkante 3.1 aufweisende Vorsprung 2.1 ist aus einer Raststellung/-position, in welcher ein Eingriff mit dem entsprechenden Hinterschnitt 1.6 erfolgt bzw. erfolgen kann (siehe Fig. 3), manuell separat, d.h. unabhängig zur Enführposition des Vorsprungs 2.1 in der Aussparung 1.5 in eine Außer-Raststellung (siehe Fig. 4) bewegbar. Hierfür sind die Vorsprünge 2.1 der Kurbel 2 gemäß dem vorliegenden Ausführungsbeispiel in dem Kurbelkopf 2.9 formschlüssig in Richtung hin zu und weg vom zugehörigen Hinterschnitt 1.6 (in radialer Richtung des Getriebe-Eingangselements 1.4) verschiebbar geführt, beispielsweise in einer Schwalbenschwanzführung (siehe Fig. 1). Dabei sei darauf hingewiesen, dass die Hinterschneidungen 1.6 jeweils zweier diametral sich gegenüberliegender Aussparungen 1.5 einander zugewandt platziert sind, d.h. die Rastkanten 3.1 der Kurbelseitigen Vorsprünge 2.1 sind voneinander in Radialrichtung abgewandt und damit den jeweilige Hinterschnitten 1.6 zugewandt, sodass bei einer (radialen) Außer-Rastbewegung der Vorsprünge 2.1 sich diese aufeinander zubewegen müssen.

Demzufolge ist ein Federelement 2.2 am Kurbelkopf 2.9 angeordnet, das zwischen zwei diametral angeordneten Vorsprüngen 2.1 eingefügt ist und diese beiden Vorsprünge 2.1 voneinander radial nach außen (in Rastposition) wegdrückt. Dabei
verfügen die Vorsprünge 2.1 im Bereich ihrer Rastkanten 3.1 auf ihrer den Hinterschneidungen 1.6 jeweils zugewandten Unterseiten über Abschrägungen oder Anfasungen 2.3, damit sie beim Einführen in die Aussparungen 1.5 des scheibenförmigen Getriebe-Eingangselements 1.4 während des axialen Abgleitens an den Hinterschneidungen 1.6 selbstständig gegen die Federkraft nach radial innen verfahren/bewegt werden und nach dem Aufsetzen der Kurbel 2 auf das scheibenförmige Getriebe-Eingangselement 1.4 in die Hinterschnitte 1.6 der Aussparungen 1.5 federvorgespannt einrasten können.

Diese formschlüssige Verrastung verhindert ein ungewolltes oder unabsichtliches Lösen der Kurbel 2 und kann durch einen alleinigen Herausziehvorgang nicht aufgehoben werden.

Zum Abnehmen beziehungsweise Lösen der Kurbel 2 von der Antriebseinheit 1.2 muss der Operateur ein Betätigungselement 2.4 an der Kurbel 2 betätigen. Dieses Betätigungselement 2.4 ist im vorliegenden Ausführungsbeispiel als Druckknopf oder Taste gestaltet und lenkt die in der Ebene des Griffhebels 3, bzw. Des Kurbelkopfs 2.9 liegende Bewegungsrichtung der Vorsprünge (Eingriffselemente) 2.1 in eine senkrecht hierzu ausgerichtete Bewegung des Druckknopfs 2.4 um. In anderen Worten ausgedrückt ist der Druckknopf 2.4 im Wesentlichen zentrisch im Kurbelkopf 2.9 zwischen den beiden Vorsprüngen 2.1 sowie auf der zu den Vorsprüngen 2.1 abgewandten Kugelkopfseite axialverschiebbar gelagert und kann somit in Längsrichtung der Vorsprünge 2.1 bewegt werden. Darüber hinaus ist der Druckknopf 2.4 mit beiden Vorsprüngen 2.1 über ein Kraftübertragungssystem wirkverbunden, wodurch die axiale Bewegung des Druckknopfs 2.4 in eine Bewegung der beiden Vorsprünge 2.1 senkrecht hierzu (d.h. in Radialrichtung) transformiert wird.

Durch Drücken dieses Druckknopfs 2.4 verfahren beide Vorsprünge 2.1 (Eingriffelemente) gegen die Federkraft gleichzeitig und symmetrisch bezüglich des scheibenförmigen Getriebe-Eingangselements 1.4 radial nach innen in die Außer-Rastposition. Nun haben sie in dieser Betätigungsstellung keinen Kontakt mehr zu den Hinterschnitten 1.6 und können durch Anheben der Kurbel 2 durch die
Aussparungen 1.5 geführt bzw. aus den Aussparungen 1.5 gezogen werden.

Das Kraft-/Bewegungsübertragungssystem ist hierfür wie folgt ausgebildet:
Der Druckknopf 2.4 hat zwei in seine Längs- bzw. Betätigungsrichtung sich erstreckende sowie in einem spitzen Winkel in Betätigungsrichtung auseinanderlaufende Abgleitkanten oder Flächen 2.6, die eine Art Führungskullisse darstellen. Des Weiteren hat jeder Vorsprung 2.1 einen Mitnehmer oder Querstift 2.5, der an der jeweils zugewandten Abgleitkante 2.6 des Betätigungsknopfs 2.4 anliegt. Wird nunmehr der Betätigungsknopf 2.4 gedrückt, gleiten die Abgleitkanten 2.6 an den jeweils anliegenden Querstiften 2.5 ab. Da die Abgleitkanten 2.6 jedoch nicht exakt parallel zur Betätigungsrichtung des Knopfs 2.4 verlaufen sondern jeweils in einem spitzen Winkel dazu, führt die Längsbewegung der Abgleitkanten 2.6 in eine diese überlagernde Querbewegung der Vorsprünge 2.1 infolge der Gleitanlage der Querstifte 2.5 an den Abgleitkanten 2.6. D.h., die Abgleitkanten 2.6 wirken funktional als Keile die auf die Vorsprünge 2.1 eine Zug- oder Druckkraft gegen die Feder 2.2 ausüben.

Mittels der Feder(n) 2.2 zwischen den Vorsprüngen (Eingriffelementen) 2.1, den Querstiften 2.5 in den Vorsprüngen (Eingriffelementen) 2.1 und den schrägen Gleitflächen 2.6 im unteren Bereich des Druckknopfs 2.4, an denen die Querstifte 2.5 anliegen, ist der Druckknopf 2.4 selbst ebenfalls federnd gelagert und fährt nach dem Betätigen wieder in seine Ausgangsstellung A gemäß der Fig. 3 zurück. Die Betätigungsstellung B gemäß der Fig. 4 kann durch Anschlag der beiden Vorsprünge (Eingriffelemente) 2.1 mittig aneinander definiert werden, das ist also der Punkt, bis zu dem der Druckknopf 2.4 gedrückt beziehungsweise vertikal verfahren werden kann.

Die Ausgangsstellung A, die aufgrund der Feder 2.2 vorliegt, wenn der Druckknopf 2.4 nicht manuell betätigt wird, muss über einen Anschlag nach oben am Druckknopf 2.4 oder einen Anschlag nach außen an den Vorsprüngen (Eingriffelementen) 2.1 definiert werden.

Eine platzsparende und kostengünstige Möglichkeit hierzu besteht darin, einen Stift 2.7 quer zum Betätigungsknopf 2.4 in den Kurbelkopf 2.9 einzuführen, der ein Langloch 2.8 des Druckknopfs 2.4 durchdringt, welches an seinen jeweiligen Langlochenden die maxiale Betätigungsposition und die Nicht-Betätigungsposition des Druckknopfs 2.4 definiert. Der Druckknopf 2.4 fährt demzufolge nach dem Betätigen federunterstützt aus dem Kurbelkopf heraus (gemäß der Fig. 3 senkrecht nach oben), bis das Ende des Langlochs 2.8 am Stift 2.7 angekommen ist. Dann ist die Ausgangsstellung hergestellt. Dieser Stift 2.7 dient auch zur Montage des Griffhebels 3 am Kurbelkopf 2.9.

An dieser Stelle seinen noch einige Abwandlungen genannt, die alternativ oder zusätzlich zu den vorstehend beschriebenen Merkmalen denkbar sind.

Das gezeigte Ausführungsbeispiel hat zwei kurbelseitige Vorsprünge von denen jeder Vorsprung mit einer Rastkante versehen ist und daher auch bewegbar am Kurbelkopf geführt sein muss. Grundsätzlich reicht es aber aus, wenn nur einer der Vorsprünge eine Rastkante hat. In diesem Fall könnte dieser eine Vorsprung vorzugsweise zentral zwischen zwei weiteren Vorsprüngen angeordnet sein und beispielsweise nur die Funktion einer Abhebsicherung übernehmen (keine Drehmomentübertragung).

Auch ist es denkbar, nicht die Vorsprünge sondern nur die Rastkante an dem betreffenden Vorsprung bewegbar zu lagern, wohingegen die Vorsprünge selbst fix am Kurbelkopf gehalten/angeformt sind. Schließlich kann anstelle einer Schiebebewegung der Vorsprünge auch eine Schwenkbewegung der Vorsprünge vorgesehen sein.

Wie eingangs bereits ausgeführt wurde, wird durch die Ausbildung von mehreren beabstandeten Aussparungen (Eingriffspunkten für die Kurbel) ein großer wirksamer Hebelarm am Getriebe-Eingangselement geschaffen, sodass die Aussparungen klein und wenig tief ausgebildet sein können und trotzdem hohe Drehmomente übertragen können. Dadurch ist es möglich, das Getriebe-Eingangselement flach, scheibenförmig zu halten. Jedoch ist es denkbar, im Fall nur einer zentralen Aussparung diese unrund zu gestalten und dadurch einen drehfesten Formschluss mit dem eingeführten Vorsprung der Kurbel zu bewirken.

Offenbart wird zusammenfassend ein Retraktor mit wenigstens zwei Retraktorarmen, deren proximale Endabschnitte an den sich gegenüberliegenden Enden eines längenverstellbaren Querriegels zur Einstellung des Querabstands zwischen den Retraktorarmen fixiert oder einstückig daran angeformt sind und einer Antriebseinheit, deren Gehäuse am Querriegel montiert ist und deren Getriebe mit einem Längenverstellmechanismus des Querriegels wirkgekoppelt ist, um mittels eines separaten Betätigungselements manuell betätigt zu werden, das mit einem in das Gehäuse bewegbar, vorzugsweise drehbar eingesetzten Getriebe-Eingangselement in Eingriff bringbar ist, das hierfür an wenigstens einer Gehäuseaußenseite von außen zugänglich ist. Erfindungsgemäß ist das Getriebe-Eingangselement so (scheibenartig) ausgebildet, dass es im Wesentlichen bündig mit der Gehäuseaußenseite abschließt oder bezüglich der Gehäuseaußenseite in das Gehäuseinnere zurückgesetzt ist. Es weist an seiner exponierten Seite Aussparungen auf, von denen wenigstens eine einen Hinterschnitt hat, der mit einem eine Rastkante ausbildenden Kraftübertragungsvorsprung des Betätigungselements bei dessen Einführen in die Aussparung in manuell lösbaren Rasteingriff für ein unbeabsichtigtes Herausziehen kommt.

## Patentansprüche

1. Retraktor mit einem separaten Betätigungselement (2) und mit wenigstens zwei Retraktorarmen (1.1), deren proximale Endabschnitte an den sich gegenüberliegenden Enden eines längenverstellbaren Querriegels zur Einstellung des Querabstands zwischen den Retraktorarmen (1.1) fixiert oder einstückig daran angeformt sind und mit einer Antriebseinheit (1.2), deren Gehäuse (1.3) am Querriegel montiert ist und deren Getriebe mit einem Längenverstellmechanismus des Querriegels wirkgekoppelt ist, um mittels des separaten Betätigungselements (2) des Retraktors manuell betätigt zu werden, das mit einem in das Gehäuse (1.3) drehbar eingesetzten Getriebe-Eingangselement (1.4) in Eingriff bringbar ist, das hierfür auf wenigstens einer Gehäuseaußenseite von außen zugänglich ist, wobei das Getriebe-Eingangselement (1.4) im Wesentlichen bündig mit dieser Gehäuseaußenseite abschließt oder bezüglich dieser Gehäuseaußenseite in das Gehäuseinnere zurückgesetzt ist und wobei
das drehbar im Getriebegehäuse (1.3) gehaltene Getriebe-Eingangselement (1.4) an seiner nach außen hin exponierten Seite wenigstens eine Aussparung (1.5) aufweist, die als ein formschlüssig wirkendes Kraft-/Momenteinleitungsmittel für das wenigstens einen Vorsprung (2.1) aufweisende Betätigungselement (2) angepasst ist, **dadurch gekennzeichnet, dass** zumindest eine Aussparung (1.5) auf Seiten der Antriebseinheit (1.2) mit einem axial wirkenden Hinterschnitt (1.6) ausgeformt ist und wenigstens ein Vorsprung (2.1) auf Seiten des Betätigungselements (2) mit einer axial wirkenden Rastkante (3.1) aufgeformt ist, derart, dass bei Einfügen des wenigstens einen Vorsprungs (2.1) in die zumindest eine Aussparung (1.5) die Rastkante (3.1) in den Hinterschnitt (1.6) greift und somit ein unbeabsichtigtes Herausziehen des Vorsprungs (2.1) aus der Aussparung (1.5) ver- oder behindert.

2. Retraktor nach Anspruch 1, **dadurch gekennzeichnet, dass** das drehbar im Getriebegehäuse (1.3) gehaltene Getriebe-Eingangselement (1.4) an seiner nach außen hin exponierten Seite eine Anzahl von in Umfangs- und/oder Radialrichtung bezüglich seiner Drehrichtung beabstandete Aussparungen (1.5) aufweist, die als Kraft-/Momenteinleitungsmittel für das Betätigungselement (2) vorgesehen sind.

3. Retraktor nach Anspruch 2, **dadurch gekennzeichnet, dass** das Betätigungselement (2) einen Hebel oder eine Kurbel hat, an dessen einem Endabschnitt eine Handhabe (3) und an dessen anderem Ende ein Kurbelkopf (2.9) vorgesehen ist mit wenigstens zwei Vorsprüngen oder Eingriffselementen (2.1), die gleichzeitig in wenigstens zwei der Aussparungen (1.5) einfügbar sind, um durch den infolge des Abstands der zumindest zwei Aussparungen (1.5) wirksamen Hebelarm ein Drehmoment auf das Getriebe - Eingangselement (1.4) zu übertragen.

4. Retraktor nach Anspruch 1, **dadurch gekennzeichnet, dass** der zumindest eine mit der Rastkante (3.1) ausgeformte Vorsprung (2.1) in und entgegen der Rastrichtung vorzugsweise quer zu seiner Längserstreckung beweg- oder verschwenkbar ist, um unabhängig von seiner Einführposition in der Aussparung (1.5) manuell zumindest in eine Außereingriffsstellung bezüglich des Hinterschnitts (1.6) überführbar zu sein.

5. Retraktor nach Anspruch 1 oder 4, **dadurch gekennzeichnet, dass** der zumindest eine mit der Rastkante (3.1) ausgeformte Vorsprung (2.1) in Rastrichtung vorgespannt oder vorspannbar ist.

6. Retraktor nach Anspruch 5, **gekennzeichnet durch** einen Betätigungsknopf oder eine Taste (2.4) am Betätigungselement (2), welche mit dem zumindest einen, eine Rastkante (3.1) aufweisenden Vorsprung (2.1) in Wirkeingriff steht.

7. Retraktor nach Anspruch 5, **dadurch gekennzeichnet, dass** wenigstens zwei mit einer Rastkante (3.1) ausgebildete Vorsprünge (2.1) vorgesehen sind, die sich bezüglich der Drehrichtung des Getriebe-Eingangselements (1.4) diametral gegenüberliegen, sodass die Rastkanten (3.1) voneinander abgewandt sind, und ein Vorspannmittel (2.2) zwischen diesen beiden Vorsprüngen (2.1) angeordnet ist, welches die beiden Vorsprünge (2.1) in eine Eingriffsstellung voneinander wegdrückt.

8. Retraktor nach Anspruch 6, **dadurch gekennzeichnet, dass** der Betätigungsknopf oder die Taste (2.4) quer zu der Bewegungsrichtung der wenigstens beiden Vorsprünge (2.1) betätigbar am Betätigungselement (2) vorzugsweise Kurbelkopf (2.9) gelagert ist und zwei in Betätigungsrichtung sich erstreckende sowie in einem spitzen Winkel voneinander sich entfernende Eingriffkanten (2.6) hat, die mit Mitnehmerzapfen (2.5) an den Vorsprüngen (2.1) in Gleitanlage sind, derart, dass bei einer Betätigung des Knopfs oder der Taste (2.4) die beiden Eingriffskanten (2.6) an den Mitnehmerzapfen (2.5) entlang gleiten und dabei infolge ihrer Winkelausrichtung die Mitnehmerzapfen (2.5) und somit die Vorsprünge (2.1) entgegen der Federvorspannung gegeneinander ziehen oder drücken.

## Claims

1. A retractor with a separate actuation element (2) and comprising at least two retractor arms (1.1) whose proximal end portions are fixed or integrally formed on the opposite ends of a longitudinally adjustable crossbar for adjusting the transverse distance between the retractor arms (1.1), and a drive gear unit (1.2) whose case (1.3) is mounted on the crossbar and whose gearing is in operative connection with a length adjustment mechanism of the crossbar in order to be manually operated by means of the separate actuation element (2) of the retractor which can be brought into engagement with a gearing input element (1.4) which is rotatably inserted in the case (1.3), and to this end is accessible from outside on at least one outer side of the case, the gearing input element (1.4) being substantially flush with said case's outer side or being recessed into the case interior with respect to said case's outer side, and the gearing input element (1.4) which is rotatably held in the gearing case (1.3) comprising at least one recess (1.5) at its face exposed towards outside, said recess being adapted to work as a force/torque introduction member, acting in form-locking manner, for the actuation element (2) comprising at least one protrusion (2.1), **characterized in that** at least one recess (1.5) on the side of the drive gear unit (1.2) is formed with an axially acting undercut (1.6) and at least one protrusion (2.1) on the side of the actuation element (2) is formed with an axially acting detent edge (3.1) such that the detent edge (3.1) engages the undercut (1.6) upon inserting the at least one protrusion (2.1) into the at least one recess (1.5) and in this way prevents/impedes the protrusion (2.1) from being unintentionally pulled out of the recess (1.5).

2. The retractor according to claim 1, **characterized in that** the gearing input element (1.4) which is rotatably held in the gearing case (1.3) comprises, at its face exposed towards outside, a number of recesses (1.5) which are spaced in the circumferential and/or radial direction with respect to its rotational direction and provided as a force/torque introduction means for the actuation element (2).

3. The retractor according to claim 2, **characterized in that** the actuation element (2) comprises a lever or a crank whose one end portion is provided with a handle (3) and whose other end is provided with a crank head (2.9) comprising at least two protrusions or engaging elements (2.1) which can be simultaneously inserted into at least two of the recesses (1.5) in order to transfer a torque to the gearing input element (1.4) by the lever arm which is effective due to the distance of the at least two recesses (1.5).

4. The retractor according to claim 1, **characterized in that** the at least one protrusion (2.1) formed with the detent edge (3.1) can be moved or swiveled preferably transverse to its longitudinal extension in and reverse to the lock-in direction, in order to be able to be manually brought at least into one disengagement position with respect to the undercut (1.6) irrespective of its insertion position in the recess (1.5).

5. The retractor according to claim 1 or 4, **characterized in that** the at least one protrusion (2.1) formed with the detent edge (3.1) is preloaded or can be preloaded in the lock-in direction.

6. The retractor according to claim 5, **characterized by** an actuator button or a key (2.4) on the actuation element (2), which is in operative connection with the at least one protrusion (2.1) comprising a detent edge (3.1).

7. The retractor according to claim 5, **characterized in that** at least two protrusions (2.1) which are formed with a detent edge (3.1) are provided and are disposed so as to be diametrically opposed with respect to the rotational direction of the gearing input element (1.4) such that the detent edges (3.1) face away from each other, and a preload means (2.2) is arranged between said two protrusions (2.1) and urges the two protrusions (2.1) away from each other to an engagement position.

8. The retractor according to claim 6, **characterized in that** the actuator button or key (2.4) is supported so as to be operable on the actuation element (2), preferably crank head (2.9), transverse to the direction of movement of the at least two protrusions (2.1) and comprises two engagement edges (2.6) extending in the direction of actuation and diverging from each other at an acute angle, said engagement edges being in sliding contact with driver pins (2.5) on the protrusions (2.1) such that the two engagement edges (2.6) slide along the driver pins (2.5) during actuation of the button or key (2.4) and due to their angular orientation pull or press the driver pins (2.5) and hence the protrusions (2.1) towards each other against the spring preload.

## Revendications

1. Rétracteur avec un élément d'actionnement séparé (2) et avec au moins deux bras de rétracteur (1.1), dont les sections d'extrémité proximales sont fixées au niveau des extrémités opposées d'une traverse ajustable en longueur pour le réglage de la distance transversale entre les bras de rétracteur (1.1) ou y sont formées d'un seul tenant et avec une unité d'entraînement (1.2), dont le boîtier (1.3) est monté au niveau de la traverse et dont la transmission est couplée activement à un mécanisme d'ajustement en longueur de la traverse, pour être actionné manuellement au moyen de l'élément d'actionnement séparé (2) du rétracteur, qui peut être mis en prise avec un élément d'entrée de transmission (1.4) inséré de manière rotative dans le boîtier (1.3), qui est accessible pour ce faire de l'extérieur sur au moins un côté extérieur de boîtier, dans lequel l'élément d'entrée de transmission (1.4) se termine sensiblement en affleurement avec ce côté extérieur de boîtier ou est replacé à l'intérieur du boîtier par rapport à ce côté extérieur de boîtier et dans lequel
l'élément d'entrée de transmission (1.4) retenu de manière rotative dans le boîtier de transmission (1.3) présente au niveau de son côté exposé vers l'extérieur au moins un évidement (1.5), qui est adapté en tant que moyen d'introduction de force/couple agissant par correspondance de forme pour l'élément d'actionnement (2) présentant au moins une saillie (2.1), **caractérisé en ce qu'**au moins un évidement (1.5) est formé sur des côtés de l'unité d'entraînement (1.2) avec une contre-dépouille (1.6) à action axiale et au moins une saillie (2.1) est formée sur des côtés de l'élément d'actionnement (2) avec un bord d'encliquetage (3.1) à action axiale, de sorte que lors de l'insertion de l'au moins une saillie (2.1) dans l'au moins un évidement (1.5), le bord d'encliquetage (3.1) vient en prise dans la contre-dépouille (1.6) et évite ou empêche ainsi un retrait involontaire de la saillie (2.1) de l'évidement (1.5).

2. Rétracteur selon la revendication 1, **caractérisé en ce que** l'élément d'entrée de transmission (1.4) retenu de manière rotative dans le boîtier de transmission (1.3) présente au niveau de son côté exposé vers l'extérieur un certain nombre d'évidements (1.5) espacés dans la direction périphérique et/ou radiale par rapport à son sens de rotation, qui sont prévus en tant que moyen d'introduction de force/couple pour l'élément d'actionnement (2).

3. Rétracteur selon la revendication 2, **caractérisé en ce que** l'élément d'actionnement (2) a un levier ou une manivelle à une section d'extrémité duquel une poignée (3) est prévue et à l'autre extrémité duquel une tête de manivelle (2.9) est prévue avec au moins deux saillies ou éléments de mise en prise (2.1), qui peuvent être introduits en même temps dans au moins deux des évidements (1.5) pour transmettre un couple à l'élément d'entrée de transmission (1.4) par le bras de levier agissant en raison de la distance des au moins deux évidements (1.5).

4. Rétracteur selon la revendication 1, **caractérisé en ce que** l'au moins une saillie (2.1) formée avec le bord d'encliquetage (3.1) est déplaçable ou pivotable dans et dans le sens opposé à la direction d'encliquetage de préférence transversalement à son extension longitudinale pour pouvoir être amenée manuellement au moins dans une position de mise en prise extérieure par rapport à la contre-dépouille (1.6) indépendamment de sa position d'introduction dans l'évidement (1.5).

5. Rétracteur selon la revendication 1 ou 4, **caractérisé en ce que** l'au moins une saillie (2.1) formée avec le bord d'encliquetage (3.1) est précontrainte ou peut être précontrainte dans la direction d'encliquetage.

6. Rétracteur selon la revendication 5, **caractérisé par** un bouton d'actionnement ou une touche (2.4) au niveau de l'élément d'actionnement (2), lequel est en prise active avec l'au moins une saillie (2.1) présentant un bord d'encliquetage (3.1).

7. Rétracteur selon la revendication 5, **caractérisé en ce qu'**au moins deux saillies (2.1) formées avec un bord d'encliquetage (3.1) sont prévues, qui sont diamétralement opposées par rapport au sens de rotation de l'élément d'entrée de transmission (1.4) de sorte que les bords d'encliquetage (3.1) sont opposés l'un à l'autre, et un moyen de précontrainte (2.2) est agencé entre ces deux saillies (2.1), lequel écarte les deux saillies (2.1) l'une de l'autre dans une position de mise en prise.

8. Rétracteur selon la revendication 6, **caractérisé en ce que** le bouton d'actionnement ou la touche (2.4) est logé transversalement à la direction de déplacement des au moins deux saillies (2.1) de manière actionnable au niveau de l'élément d'actionnement (2) de préférence de la tête de manivelle (2.9) et a deux bords de mise en prise (2.6) s'étendant dans la direction d'actionnement et s'éloignant l'un de l'autre selon un angle aigu, qui sont logés en appui coulissant avec des broches d'entraînement (2.5) au niveau des saillies (2.1) de sorte que lors d'un actionnement du bouton ou de la touche (2.4) les deux bords de mise en prise (2.6) glissent le long des broches d'entraînement (2.5) et tirent ou poussent ce faisant en raison de leur orientation angulaire les broches d'entraînement (2.5) et ainsi les saillies (2.1) l'une contre l'autre contre la précontrainte de ressort.
